# EUROPEAN PATENT APPLICATION

(11) **EP 1 818 336 A1**
(43) Date of publication of application: **15.08.2007**
(21) Application number: 06021480.6
(22) Date of filing: 13.10.2006
(51) Int. Cl.: C07F 9/54, C07D 233/54, C07C 211/63

(54) **2,2-bis-(4-hydroxyphenyl)-alkyl onium salt and a process for the preparation thereof**

(30) Priority: 13.02.2006 IN DE04002006
(71) Applicant: Council of Scientific and Industrial Research, New Delhi - 110 001 (IN)
(72) Inventor: Shroff, Rama Mallikarjuna National Chem. Labor., Purna-411 008 Maharashtra (IN); Swaminathan, Sivaram National Chemical Laboratory, Purna-411 008 Maharashtra (IN)
(74) Representative: Allsop, John Rowland

(57) **Abstract**

The present invention provides a novel 2,2-bis(4-hydroxyphenyl)-alkyl onium salts as illustrated by 2,2-bis(4-hydroxyphenyl)-tridecyl(1,2-dimethylimidazolium) bromide and the process for preparation thereof by hydroxyalkylating acetoacetate to the corresponding hydroxyalkylacetoacetate, dealkoxycarbonylating the hydroxyalkyl acetoacetate to ω-hydroxyalkan-2-one and contacting the ω-hydroxyalkan-2-one with phenol in the presence of an acidic catalyst to give 2,2-bis(4-hydroxyphenyl)alkanol, brominating the 2,2-bis(4-hydroxyphenyl)alkanol to 2,2-bis(4-hydroxyphenyl)alkyl bromide, quaternizing 1,2-dimethylimidazole with the 2,2-bis(4-hydroxyphenyl)alkyl bromide to 2,2-bis(4-hydroxyphenyl)alkyl(1,2-dimethylimidazolium) bromide. The products can be used as reactive modifiers for layered phyllosilicates that can be used in the preparation of polymer-nanocomposites, wherein the said polymers are prepared from 2,2-bis(4-hydroxyphenyl)propane as one of the reacting monomers.

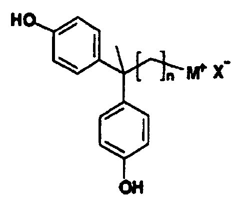

## Description

**Field of the invention** The present invention relates to 2,2-bis(4-hydroxyphenyl)-alkyl onium salts, and process for preparation thereof. More particularly it relates to the said salts of formula (1) 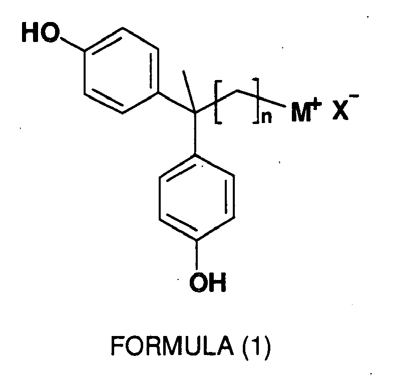 Wherein M= trialkylphosphonium , triarylphosphonium, triarylalkylphosphonium or substituted cylic amidinium radical, n=1 to 36 and X= Cl, Br, I, BF₄, OTf, or NTf₂,

### Background of the invention

The organophilic phyllosilicates can be used as fillers for thermoplastic materials and also for thermosets, giving nanocomposites. When suitable organophilic phyllosilicates are used as fillers, the physical and mechanical properties of the moldings thus produced are considerably improved. A particularly interesting feature is the.increase in stiffness with no decrease in toughness. Nanocomposites, which comprise the phyllosilicate in exfoliated form, have particularly good properties

The product of this invention may be used to exchange monovalant cations present in the layered phyllosilicates and hence modify the hydrophilic phyllosilicates into organophilic phyllosilicates capabale of anchoring the polymer chain more preferably polyether ether ketones, polysulfones, polyethersulfones, polycarbonates, polyarylates, epoxy etc. during preparation of phyllosilicate/polymer nanocomposites via in-situ polymerization.

U.S. Pat. No. 4,810,734 has disclosed that phyllosilicates can be treated with a quaternary or other ammonium salt of a primary, secondary or tertiary linear organic amine in the presence of a dispersing medium. During this process there is ion exchange or cation exchange, where, the cation of the ammonium salt becomes embedded into the space between the layers of the phyllosilicate. The organic radical of the absorbed amine makes phyllosilicates modified in this way organophilic. When this organic radical comprises functional groups the organophilic phyllosilicate is able to enter into chemical bonding with a suitable monomer or polymer.

There are many examples in the patent literature wherein the preparation of polymer/clay nanocomposites from monomers and treated clays has been described: For example, U.S. Pat. No. 4,739,007 discloses the preparation of Nylon-6/clay nanocomposites from caprolactam and alkyl ammonium-treated montmorillonite.

However the above-mentioned phyllosilicates modified with alkylammonium cations undergo degradation at the temperatures above 250 °C as illustrated in the published literature, Xie et. al., Chemistry of Materials, 2001 13, 2979 -2990, the disclosures of which are incorporated by reference herein. The decomposition of these modifiers during the preparation or processing of the nanocomposites leads to degradation of the polymers by chain scission reactions, color formation etc. The formation of decomposition products can lead to emissions and to impairment of mechanical properties such as impact strength. Thermostable modifier treatment is required in the preparation of polymer/clay nanocomposites wherein the polymer resins such as polycarbonates, polyethylene terepthalate, or any other polymers are used having processing temperatures above the 250 °C, The use of thermally stable modifiers based on cyclic amidinium ions is disclosed in the US. Pat. Nos. 5530052, 5707439, 6197849, 20040033392A1, the disclosure of which are incorporated by reference herein.
US. Patents Nos. 6057035, 6287992, 6262162, 6359052, teach the use of thermostable modifiers based on phosphonium ions. However the dispersion of layered phyllosilicates, which are modified with such amidinium or phosphonium based surfactants, in the polymer resin was poor and always resulted in intercalated nanocomposites wherein the clay platelets still remained intact and polymer chains have intercalated in between the clay gallery and increase the interlayer distance.
Polycarbonate nanocomposites, as disclosed in US. Pat. Nos. 2004/0030021 A1, have resulted in only intercalated nanocomposites and sometimes decrease in interlayer distance for the phyllosilicate due to de-intercalation of the modifier itself.

It is observed that maximum improvement in the properties can be exploited only when the platelets are completely delaminated/exfoliated in the polymer resin matrix. It is also observed that when the modifier is capable of anchoring the polymer chains will enhance interaction of the polymer resin with the phyllosilicate layered platelets and under suitable process in making the nanocomposite result in fully exfoliated nanocomposites.

### Objectives of the invention

The main object of the present invention is to provide 2,2-bis(4-hydroxyphenyl)-alkyl onium salts, and process for preparation thereof.

Another object of the present invention is to provide a suitable modifier for the phyllosilicates such that it can be used in the preparation of clay polymer nanocomposites, wherein the polymer can be from a variety groups such as poly(ether ether ketones), polysulfones, polyethersulfones, polycarbonates, polyarylates and epoxy resins.

Yet another object of the present invention is to provide a modifier for phyllosilicates, which change hydrophilic phyllosilicates into organophilic.

Yet another object of the present invention is to provide a modifier for the phyllosilicate, which are stable at the temperatures of preparation and processing of polymer-nanocomposites, wherein the said polymer is made from 2,2-bis(4-hydroxyphenyl)propane as one of the reacting monomers.

Yet another object of the present invention is to provide a modifier for the phyllosilicate such that it contains reactive moiety that can anchor the polymer chains covalently on to the phyllosilicate- layers, wherein the said polymer is made from 2,2-bis(4-hydroxyphenyl)propane as one of the reacting monomers.

Yet another object of the present invention is to provide a method for the preparation of the modifier for the phyllosilicate, which can change hydrophilic phyllosilicates into organophilic, stable at temperatures of preparation and processing of polymer-nanocomposites, and comprises a reactive moiety, which can anchor polymer chains on to the phyllosilicate layers, wherein the said polymer is made from 2,2-bis(4-hydroxyphenyl)propane as one of the reacting monomers.

### Summary of the invention

Accordingly the present invention provides a novel 2,2-bis(4-hydroxy phenyl) alkylonium salts of formula (1) 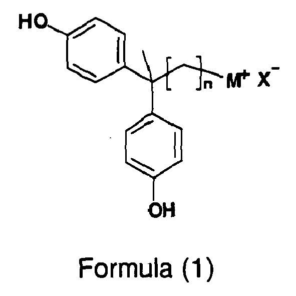 wherein n=1 to 37, X= CI, Br, I, BF₄, OTf, or NTf₂, M=trialkylphosphonium, triarylphosphonium, triaryl- alkylphosphonium, ammonium or substituted cylic amidinium radical selected from the group consisting of pyrrole, imidazole, thiazole, oxazole, pyridine, pyrimidine, quinoline, isoquinoline, indole, purine, benzimidazole, benzothiazole, benzoxazole, pyrazine, quinoxaline, quinozoline, acridine, phenazine, imidazopyridine and dipyridyl.

In an embodiment of the present invention the 2,2-bis(4-hydroxy phenyl) alkylonium salt is 2,2-bis(4-hydroxyphenyl)-tridecyl(1,2-dimethylimidazolium) bromide

The present invention further provides a process for the preparation of 2,2-bis(4-hydroxyphenyl)alkyl onium salts of formula (1) which comprises
a) Mono brominating alkyl dialcohol by reacting it with a brominating agent in an organic solvent, at a temperature in the range of 40-150°C to obtain α ω-bromoalkylalcohol of formula (2), 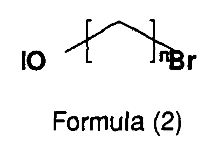
b) alkylating acetoacetate with α,ω-bromoalkylalcohol of formula (2)in dry organic solvent in the presence of base and accelerating agent, at a temperature in the range of 0-80°C to obtain hydroxy alkyl acetoacetate of formula (3), 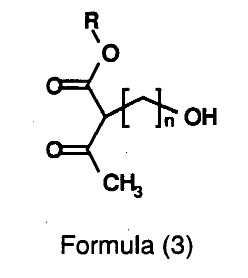 wherein R= CH₃, C₂H₅, and n= 1 to 36
c) dealkoxycarbonylating the compound of formula (3) either by first hydrolysing the compound of formula (3), followed by decarboxylation or in a single step dealkoxycarbonylation by adding alkali metal salt or ammonium salt along with equivalent amount of water to a non aqueous solution of compound of formula (3), at a temperature in the range of 40-250°C, cooling the above said reaction mixture to a temperature of 20-30°C, and pouring it into water, followed by extraction with diethyl ether and washing with water, brine and finally drying the resultant product to obtain ω-hydroxyalkan-2-one of formula (4), 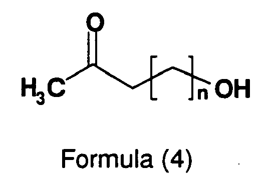 wherein n= 1 to 37,
d) reacting the compound of formula (4) with phenol in presence of acidic catalyst, under stirring, at a temperature in the range of 10 °C to 60 °C, for a period of 10-15 hrs, dissolving the resultant reaction mixture in ethyl acetate followed by washing with water, NaHCO₃ and brine and finally drying and removing the excess phenol under vacuum at about 60°C to obtain 2,2-bis(4-hydroxyphenyl)alkanol of the formula (5), where n= 1 to 37 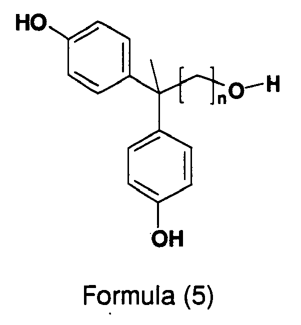
e) brominating the compound of formula (5) by reacting with a brominating agent in dry organic solvent, at a temperature in the range of -10 to 100°C, under stirring to obtain 2,2-bis(4-hydroxyphenyl) alkyl bromide of the formula (6), 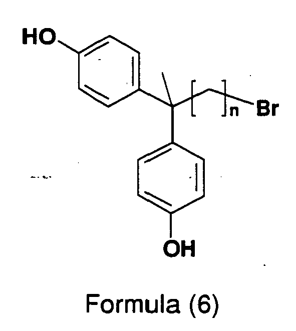
f) alkylating compound of formula (6) by reacting it with an alkylating reagent in presence of organic solvent, at a temperature in the range of 35-110°C to obtain the salts of the formula (1).

In yet another embodiment the alkyl dialcohol used in step (a) is 1, 10 decanediol.

In yet another embodiment the α,ω-bromoalkylalcohol obtained is 10-bromodecan-1-ol.

In yet another embodiment the organic solvent used in step (a) used is selected from the group consisting of toluene, benzene, cyclohexane, ethoxyethane and tetrahydrafuran.

In yet another embodiment the brominating agent used in step (a) is selected from the group consisting of aqueous HBr, HBr (phase transfer), HBr/H₂SO₄, HBr/LiBr, (C5H5N⁺H)F(HF)⁻X/NH₄Br, ZnBr₂/PPh₃/EtO₂CN=N CO₂Et, PBr₃, P/Br₂, Ph₃P/Br₂, Ph₂PCH₂CH₂PPh₂/Br₂, nBu₃P/Br₂, (PhO)₃P /Br₂, (PhO)₃P /C₆H₅CH₂Br, CBr₄/PPh₃, Ph₃P/NBS, Ph₂PCH₂CH₂PPh₂/CBr₄, Ph₃P/BrCl₂CCCl₂Br, SOBr₂, Me₂S/NBS, BH₃/Br₂, (Me₃Si)₂/(C₅H₅NH)Br₃, Me₃SiCl/LiBr and Me₃SiBr.

In yet another embodiment the monoalkylation at active methylene carbon of acetoacetate in step (b) is carried out by generating an anion at that carbon using a base selected from the group consisting of sodium methoxide, sodium ethoxide, NaH and K₂CO₃.

In yet another embodiment the organic solvent used in step (b) is alcohol selected from the group consisting of methanol, ethanol, isopropanol, isobutanol, tetrahydrafuran, diethyl ether and hydrocarbon selected from cyclohexane, hexane, pet ether and decalin.

In yet another embodiment the accelerating agent used in step (b) is selected from tetra n-butylammonium iodide, sodium iodide and potassium iodide.

In yet another embodiment the alkali metal salt used in step (C) is selected from the group consisting of NaCl, NaBr, KCl, KBr, Nal, Kl, NaCN, LiCl, Lil and KOAc.

In yet another embodiment the ammonium salt used in step (C) is (H₃C)₄NOAc.

In yet another embodiment the non-aqueous solvent used in step (c) is selected from the group consisting of dimethylsulfoxide, dimethyl acetate, dimethylformamide and Hexamethylphosphoric triamide.

In yet another embodiment the acidic catalyst in step (d), is selected from the group consisting of ion exchange resins in the acid form, acidic clays, sulfonated zirconia and excess of anhydrous hydrogen chloride in combination with a mercaptan such as mercaptopropionic acid.

In yet another embodiment the brominating agent used in step (e) is aqueous HBr, HBr (phase transfer), HBr/H₂SO₄, HBr/LiBr, (C5H5N⁺H)F(HF)⁻X/NH₄Br, ZnBr₂/PPh₃/EtO₂CN=NCO₂Et, PBr₃, P/Br₂, Ph₃P/Br₂, Ph₂PCH₂CH₂PPh₂/Br₂, nBu₃P/Br₂, (PhO)₃P/Br₂, (PhO)₃P /C₆H₅CH₂Br, CBr₄/PPh₃, Ph₃P/NBS, Ph₂PCH₂CH₂PPh₂/CBr₄, Ph₃P/BrCl₂CCCl₂Br, SOBr₂, Me₂S/NBS, BH₃/Br₂, (Me₃Si)₂/(C₅H₅NH)Br₃,Me₃SiCl/LiBr and Me₃SiBr.

In yet another embodiment the amount of brominating agent used in step (e) is in slight excess over the stoichiometric amount by 5 to 25 %.

In yet another embodiment the alkylation in in step (f) is done by reacting the compounds selected from the group consisting of tertiary amines, phosphines, imidazoles and pyridines with the 2,2-(bishydroxyphenyl)alkyl bromide.

In still another embodiment the organic solvent used in step (f) is selected from the group consisting of methanol, ethanol, isopropanol, butanol, isobutanol, toluene, benzene and tetrachloroethane.

The process of the present invention is described with reference to the examples hereinbelow which are illustrative only and should not be construed to limit the scope of the present invention in any manner.

### EXAMPLE 1

To a mixture of 1,10-decanediol (35.73g, 0.205 mol) and toluene (700 mL) was added concentrated HBr (29 mL of 47% aqueous solution, 0.24 mol). The heterogeneous mixture was stirred and heated at reflux for 36 hours. TLC analysis indicated substantial amounts of 1,10-decanediol still remained. Thus a further quantity of HBr (15 mL, 0.12 mol) was added and the mixture was heated at reflux for further 36 h, at which time TLC analysis showed no diol remaining. The reaction mixture was allowed to cool to room temperature and the phases were separated. The organic layer was concentrated by evaporating the toluene and diluted with ethyl acetate and washed with water, sodium bicarbonate and brine. Then the organic layer was dried over Na₂SO₄ and concentrated to yellow liquid and purification of this crude reaction mixture by column chromatography provided pure 10-bromodecanol (43.0 g) in 90% yield.

### EXAMPLE 2

Clean dry sodium (9.5 g, 0.413 mol) was placed in a three neck round bottomed flask fitted with double surface codenser, dropping funnel and septum adapter. Dry methanol (200 mL) was added on sodium slowly under cooling. Methyl acetoacetate (48.3 g, 0.416 mol) was added under stirring and heated to gentle heating. Kl (5.6 g, 0.033 mol) was added. Then 10-bromodecan-1-ol (79 g, 0.33 mol) was taken in dropping funnel and added slowly into the contents of the round bottomed flask over a period of 60 min. Continued reflux for 12 hours and monitored the reaction by TLC. The reaction was stopped when all the bromodecanol was consumed. The crude reaction mixture was concentrated by evaporating methanol, diluted with ethyl acetate, washed with water several times until the washings was neutral to litmus. Pure mehyl-(3-hydroxydecyldecyl)acetoacetate (58.3 g) was separated from crude by flash chromatography in 65% yield.

### EXAMPLE 3

Hydroxyketoester (54.4 g, 0.20 mol) was dissolved in dimethyl sulfoxide (150 mL) in a round bottom flask and NaCl (15 g, 0.25 mol) was added to it along with distilled water (18 g, 1.0 mol). The above mixture was heated at 150 °C for 18 hours. The reaction was continued until all the starting material was consumed which was monitored by TLC. Cooling it to room temperature stopped the reaction. Then it was poured into water and extracted with diethyl ether. The combined ether layer was washed with water, brine and then dried over sodium sulfate. Then the ether was evaporated to get 13-hydroxytridecan-2-one (32.1 g) as a white solid, which is then purified by recrystallization in hot petroleum ether. Yield: 75 %.

### EXAMPLE 4

13-hydroxytridecan-2-one (21.4 g, 0.10 mol) was mixed with phenol (56.4 g, 0.60 mol) and mercaptopropionic acid (0.106 g, 0.010 mol) was added to it. The anhydrous HCl gas was passed to the reaction mixture through a bubbler. The reaction was continued under stirring for 12 hours at 45 °C. Then the reaction mixture was dissolved in ethyl acetate and was washed with water, NaHCO₃ and then brine. The organic layer was dried over Na₂SO₄. The excess phenol in the reaction mixture was distilled out under vacuum at 60 °C. The 2,2-bis(4-hydroxyphenyl)tridecanol (25.0 g) was isolated by column chromatography. Yield: 65 %).

### EXAMPLE 5

The 2,2-bis(4-hydroxyphenyl)tridecanol (22.37 g, 0.050 mol) and CBr₄ (19.92g, 0.060 mol) was dissolved in dry tetrahydofuran (100mL) and taken in three-neck round bottomed flask fitted with a dropping funnel, a condenser and a three-way stopcock. Triphenylphosphine (14.41g, 0.055 mol) was dissolved in tetrahydofuran and added to the reaction mixture, which is kept at 0 °C slowly, drops by drop through a dropping funnel for duration of 30 minutes. The stirring was continued for another 4 hours at 0 ° C. The reaction was monitored by TLC. After the reaction was over, the crude reaction mixture was concentrated by evaporating tetrahydrofuran and dissolved in ethyl acetate, washed with water. The 2,2-bis(4-hydroxyphenyl)tridecyl bromide (20.1 g) was isolated in pure form after column chromatography. Yield: 90 %.

### EXAMPLE 6

Equivalent amounts of 2,2-bis-(4-hydroxyphenyl)tridecyl bromide (4.4746 g, 0.010 mol) and 1,2-dimethylimidazole (0.9613g, 0.010 mol) were mixed and heated at 100°C for 8 hours under nitrogen atmosphere. The melted mixture solidified after the reaction. 2,2-bis-(4-hydroxyphenyl)tridecyl-(1,2-dimethylimidazolium) bromide was obtained in pure form and used without further purification.

### The main advantages of the present invention are:

1. The present invention provides a novel 2,2-bis(4-hydroxyphenyl)-alkyl onium salts, a simple method for the synthesis of the same.
2. Such novel 2,2-bis(4-hydroxyphenyl)alkylonium ions may find application as modifiers for the phyllosilicates to use them in the preparation of polymer nanocomposites.
3. Such a class of novel 2,2-bis(4-hydroxyphenyl)alkylonium salts may also find applications as antistatic agents, antimicrobial agents, when suitably incorporated in polymers which have 2,2-bis(4-hydroxyphenyl)propane as one of the reacting monomers.
4. The method of the invention is simple and easy to work up.

## Claims

1. A novel 2,2-bis(4-hydroxyphenyl)alkylonium salts of formula (1) 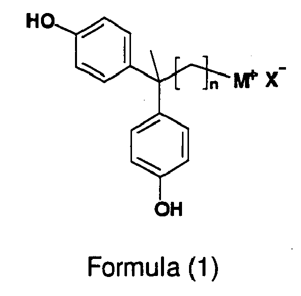 wherein n=1 to 37, X= Cl, Br, I, BF₄, OTf, or NTf₂, M=trialkylphosphonium, triarylphosphonium, triaryl- alkylphosphonium, ammonium or substituted cylic amidinium radical selected from the group consisting of pyrrole, imidazole, thiazole, oxazole, pyridine, pyrimidine, quinoline, isoquinoline, indole, purine, benzimidazole, benzothiazole, benzoxazole, pyrazine, quinoxaline, quinozoline, acridine, phenazine, imidazopyridine and dipyridyl.

2. A process for the preparation of 2,2-bis(4-hydroxyphenyl)alkyl onium salts of formula (1), the said process comprises the steps of:
a) mono brominating alkyl dialcohol by reacting it with a brominating agent in an organic solvent, at a temperature in the range of 40-150°C to obtain α ω-bromoalkylalcohol of formula (2), 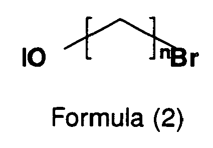
b) alkylating acetoacetate with α,ω-bromoalkylalcohol of formula (2) in dry organic solvent in the presence of base and accelerating agent, at a temperature in the range of 0-80°C to obtain hydroxy alkyl acetoacetate of formula (3), 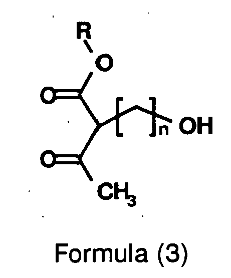 wherein R= CH₃, C₂H₅, and n= 1 to 36
c) dealkoxycarbonylating the compound of formula (3) either by first hydrolysing the compound of formula (3), followed by decarboxylation or in a single step dealkoxycarbonylation by adding alkali metal salt or ammonium salt along with an equivalent amount of water to a non aqueous solution of compound of formula (3), at a temperature in the range of 40-250°C, cooling the above said reaction mixture to a temperature of 20-30°C, and pouring it into water, followed by extraction with diethyl ether and washing with water, brine and finally drying the resultant product to obtain ω-hydroxyalkan-2-one of formula (4), 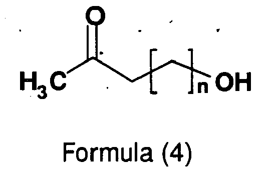 wherein n= 1 to 37,
d) reacting the compound of formula (4) with phenol in presence of acidic catalyst, under stirring, at a temperature in the range of 10 °C to 60 °C, for a period of 10-15 hrs, dissolving the resultant reaction mixture in ethyl acetate followed by washing with water, NaHCO₃ and brine and finally drying and removing the excess phenol under vacuum at about 60°C to obtain 2,2-bis(4-hydroxyphenyl)alkanol of the formula (5), where n= 1 to 37 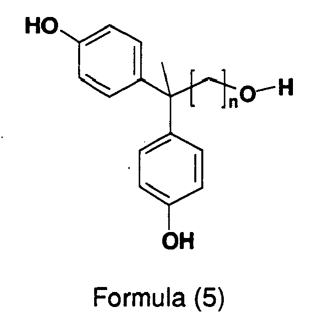
e) brominating the compound of formula (5) by reacting it with a brominating agent in dry organic solvent, at a temperature in the range of -10 to 100°C, under stirring, to obtain 2,2-bis(4-hydroxyphenyl) alkyl bromide of the formula (6), 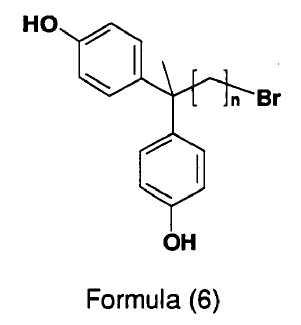
f) alkylating compound of formula (6) by reacting it with an alkylating reagent in presence of organic solvent, at a temperature in the range of 35-110°C to obtain the salts of the formula (1).

3. A process according to claim 2 wherein the alkyl dialcohol used in step (a) is 1,10 decanediol.

4. A process according to claim 2 wherein the α,ω-bromoalkylalcohol obtained is 10-bromodecan-1-ol.

5. A process according to claim 2 wherein the organic solvent used in step (a) used is selected from the group consisting of toluene, benzene, cyclohexane, ethoxyethane and tetrahydrafuran.

6. A process according to claim 2 wherein brominating agent used in step (a) is selected from the group consisting of aqueous HBr, HBr (phase transfer), HBr/H₂SO₄, HBr/LiBr, (C5H5N⁺H)F(HF)⁻X/NH₄Br, ZnBr₂ /PPh₃/EtO₂CN=N CO₂Et, PBr₃, P/Br₂, Ph₃P/Br₂, Ph₂PCH₂CH₂PPh₂/Br₂, nBu₃P/Br₂, (PhO)₃P /Br₂, (PhO)₃P /C₆H₅CH₂Br, CBr₄/PPh₃, Ph₃P/NBS, Ph₂PCH₂CH₂PPh₂/CBr₄, Ph₃P/BrCl₂CCCl₂Br, SOBr₂, Me₂S/NBS, BH₃/Br₂, (Me₃Si)₂/(C₅H₅NH)Br₃. Me₃SiCl/LiBr and Me₃SiBr.

7. A process according to claim 2 wherein the monoalkylation at active methylene carbon of acetoacetate in step (b) is carried out by generating an anion at that carbon using a base selected from the group consisting of sodium methoxide, sodium ethoxide, NaH and K₂CO₃.

8. A process according to claim 2 wherein the organic solvent used in step (b) is alcohol selected from the group consisting of methanol, ethanol, isopropanol, isobutanol, tetrahydrafuran, diethyl ether and hydrocarbon selected from cyclohexane, hexane, pet ether and decalin.

9. A process according to claim 2 wherein the accelerating agent used in step (b) is selected from tetra n-butylammonium iodide, sodium iodide and potassium iodide.

10. A process according to claim 2 wherein the alkali metal salt used in step (C) is selected from the group consisting of NaCl, NaBr, KCI, KBr, Nal, Kl, NaCN, LiCl, Lil and KOAc.

11. A process according to claim 2 wherein the ammonium salt used in step (C) is (H₃C)₄NOAc.

12. A process according to claim 2 wherein the non aqueous solvent used in step (c) is selected from the group consisting of dimethylsulfoxide, dimethyl acetate, dimethylformamide and Hexamethylphosphoric triamide.

13. A process according to claim 2 wherein the acidic catalyst in step (d), is selected from the group consisting of ion exchange resins in the acid form, acidic clays, sulfonated zirconia and excess of anhydrous hydrogen chloride in combination with a mercaptan such as mercaptopropionic acid.

14. A process according to claim 2 wherein the brominating agent used in step (e) is selected from the group consisting of aqueous HBr, HBr (phase transfer), HBr/H₂SO₄, HBr/LiBr, (C5H5N⁺H)F(HF)X/NH₄Br, ZnBr₂/PPh₃/EtO₂CN=NCO₂Et, PBr₃, P/Br₂, Ph₃P/Br₂, Ph₂PCH₂CH₂PPh₂/Br₂, nBu₃P/Br₂, (PhO)₃P/Br₂, (PhO)₃P /C₆H₅CH₂Br, CBr₄/PPh₃, Ph₃P/NBS, Ph₂PCH₂CH₂PPh₂/CBr₄, Ph₃P/BrCl₂CCCl₂Br, SOBr₂, Me₂S/NBS, BH₃/Br₂, (Me₃Si)₂/(C₅H₅NH)Br₃, Me₃SiCl/LiBr and Me₃SiBr.

15. A process according to claim 2 wherein the amount of brominating agent used in step (e) is in slight excess over the stoichiometric amount by 5 to 25 %.

16. A process according to claim 2 wherein the alkylation in step (f) is done by reacting the compounds selected from the group consisting of tertiary amines, phosphines, imidazoles and pyridines with the 2,2-bis-4-(hydroxyphenyl)alkyl bromide.

17. A process according to claim 2 wherein the organic solvent used in step (f) is selected from the group consisting of methanol, ethanol, isopropanol, butanol, isobutanol, toluene, benzene and tetrachloroethane.
